# EUROPEAN PATENT APPLICATION

(11) **EP 2 260 838 A1**
(43) Date of publication of application: **15.12.2010**
(21) Application number: 10008615.6
(22) Date of filing: 20.11.2002
(51) Int. Cl.: A61K 31/00, A61K 31/131, A61K 31/4015, A61K 45/06, A61L 27/54, A61P 9/00, A61P 43/00, A61K 31/17, A61K 31/7036, A61K 31/785, A61L 31/10, A61L 31/16

(54) **Drug for preventing vascular restenosis and instrument to be embedded in vessel coated with the drug**

(30) Priority: 20.11.2001 JP 2001354931; 12.11.2002 JP 2002328138
(62) Divisional of application: 02781819.4
(71) Applicant: NIPRO CORPORATION, Kita-ku, Osaka-shi, Osaka-fu, 531-8510 (JP); K.I.C. Co., Ltd., Yokohama-shi, Kanagawa 236-0038 (JP)
(72) Inventor: Ikari, Yuji, Suginami-ku Tokyo 167-0041 (JP)
(74) Representative: Baldock, Sharon Claire

(57) **Abstract**

A drug for preventing vascular restenosis comprises a compound that inactivates α1-protease inhibitor and α2-macroglobulin as active constituents, which are associated with occurrence of vascular restenosis after coronary artery intervention. Preferably, the drug is coated on a surface of an implantable intravascular device and locally administered to the affected area by application of the implantable intravascular device to a narrowed area.

## Description

### Technical Field

The present invention relates to a vascular restenosis preventing agent and an implantable intravascular device coated therewith. More particularly, the invention relates to a drug for preventing vascular restenosis after coronary intervention, and an implantable intravascular device coated therewith.

### Background Art

In late years, as a therapy for ischemic heart diseases resulting from coronary artery stenosis such as angina pectoris and myocardial infarction, a coronary intervention has been popularized rapidly in place of highly invasive coronary artery bypass graft surgery.

Typical coronary artery intervention is a percutaneous transluminal coronary angioplasty (PTCA) in which a lumen of a blood vessel is expanded by inserting a catheter with an inflatable balloon at a distal end thereof and then inflating the balloon at a stenosed area of a coronary artery. However, the therapy only by PTCA provides postoperative restenosis at a high frequency of about 40%, which has need of another therapy, causing the patient great mental and physical sufferings.

In order to solve such problems, a procedure, called stent placement, of placing a stent or a coiled or meshed cylindrical support of a metal or plastic at a widened stenosed area, has widely been brought into effect. This procedure has reduced the vascular restenosis rate to the halves.

The stent placement enables to prevent elastic recoil and vascular remodeling, but it is difficult to completely prevent the patient from vascular restenosis in the chronic stage. In fact, the stent placement provides about 20% probabilities of occurrence of an In-Stent Restenosis (ISR) after treatment. In particular, diabetic patients have higher frequencies of ISR.

It is conceived that the occurrence of ISR results from inflammation of the vessel walls caused by stent replacement, which causes intimal hyperplasia resulting from migration of smooth muscle cells from media to the intima.

For that reason, recent studies of the prevention of ISR have focused on inhibition of the neointimal hyperplasia, suggesting usefulness of drugs such as tranilast, paclitaxel, probucol, cilostazol and rapamycin. Some therapies employing stents coated with such a drug have been reported in Japanese unexamined patent publications such as, for example, JP2000-95706 (cf. page 3) and JP2000-249709 (cf. page 3). However, there is no generally accepted view that these drugs have remarkable effects on the prevention of ISR.

On the other hand, radiotherapies irradiating the coronary artery disease sites with β-rays or γ-rays to inhibit the neointimal hyperplasia have been put into practical use with good results. However, the radiotherapy has not yet been popularized since it causes some complications such as edge effects or late thrombosis and is difficult to control an exposed dose and radiation source.

In the current status, the prevention of vascular restenosis after coronary artery intervention is waiting for development of drugs or medical devices, which have fully satisfying effects on prevention of vascular restenosis after coronary artery intervention.

### Disclosure of Invention

The inventors have studied solution of the above problems and found that the mechanism of ISR begins with migration of smooth muscle cells to fibrin adhered to the stent surfaces, and that α₁-protease inhibitor, α₁-antichymotrypsin and α₂-macroglobulin in serum act as significant related factors in the initial stage where the fibrins adhere to the stent surface. These studies are reported in papers (Yuji Ikari et al, JOURNAL OF BIOLOGICAL. CHEMISTRY, Vol.275, Issue 17, 12799-12805, April, 2000: Yuji Ikari et al, JOURNAL OF BIOLOGICAL CHEMISTRY Vol.276, Issue 15, 11798-11803, April, 2001). At the time of announcement of the papers, it was not clear what factor is an important factor related to ISR and thus there is no conclusion on the ISR-relating factors.

Farther studies based on the above findings have been made on relationship between ISR and the level of the above factors in the blood for the patients treated by the stenting procedure. AS a result, the inventors have concluded that the α₁-protease inhibitor and α₂-macroglobulin are related to narrowing of the blood vessel, but α₁-antichymotrypsin has no relationship to the narrowing. The inventors have further studied on the above finding and achieved the present invention on the finding that ISR can be prevented effectively by deactivating α₁-protease inhibitor and α₂-macroglobulin with a certain substance.

According to the present invention, there is provided a drug for preventing vascular restenosis, which comprises, as an active constituent, a compound that deactivates α₁-protease inhibitor.

The above compound that deactivates α₁-protease inhibitor is a compound, or a salt thereof, represented by the general formula: 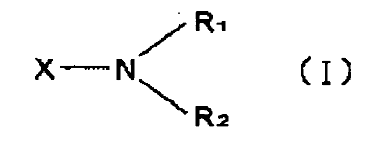 wherein X is halogen, R₁ is hydrogen or an alkaline metal, R₂ is hydrogen or arylsulfonyl which may be being substituted, R₁ and R₂ taken together, can form a group represented by the general formulas:

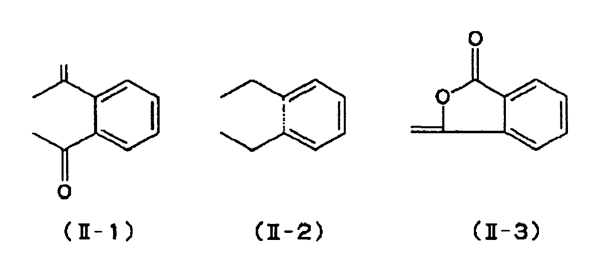

or -CO-R₃-CO- (II-4)

wherein R₃ is a divalent group or bond of linear or branched C₁ - C₅ hydrocarbons which may have one or more unsaturated bonds which may have one or more unsaturated bonds.

In the above general formula, halogen atom expressed by X includes, for example, fluorine atom, chlorine atom, bromine atom and iodine atom, and a preferred halogen atom is chlorine atom. Examples of the alkaline metal represented by R₁ are lithium, sodium and potassium, but a preferred alkaline metal is sodium.

The arylsulfonyl represented by R₂ may have 1-5 substituents, preferably, 1-3 substituents, which may be the same or different with one another, at substitutable positions thereof. The arylsulfonyl includes phenylsulfonyl, naphtylsulfonyl and the like. As preferred examples of the above substituents, there may be used, for example, C₃-C₁₀ cycloalkyl such as cyclopropyl, cyclobutyl, cyclopentyl and cyclohexyl; C₂-C₁₀ alkenyl such as vinyl, allyl, 2-methylallyl, 2-butenyl, 3-butenyl, 3-octenyl; C₂-C₁₀ alkynyl such as ethynyl, 2-propynyl, 3-hexynyl; C₃-C₁₀ cycloalkenyl such as cyclopropenyl, cyclopentenyl and cyclohexenyl; C₆-C₁₀ aryl such as phenyl and naphthyl; C₇-C₁₁ aralkyl such as benzyl and phenylethyl; nitro; nitroso; hydroxyl; mercapto; cyano; oxo; thioxo; carbamoyl; mono or di- C₁-C₆ alkylcarbamoyl such as methylcarbamoyl, dimethylcarbamoyl; C₆-C₁₄ arylcarbamoyl such as phenylcarbamoyl; carboxyl; C₁-C₄ alkoxycarbonyl such as methoxycarbonyl, ethoxycarbonyl; C₆-C₁₄ aryloxycarbonyl such as phenoxycarbonyl; sulfo; halogen such as fluorine, chlorine, bromine and iodine; C₁-C₄ alkoxy such as methoxy, ethoxy, propoxy, isopropoxy, butoxy, isobutoxy, sec-butoxy, tert-butoxy; C₆-C₁₀ aryloxy such as phenoxy; C₁-C₄ alkylthio such as methylthio, ethylthio, propylthio, isopropylthio, butylthio, isobutylthio, sec-butylthio, tert-butylthio; C₆-C₁₀ arylthio such as phenylthio; C₁-C₄ alkylsulfinyl such as methylsulfinyl, ethylsulfinyl, propyl-sulfinyl, isopropylsulfinyl, butylsulfinyl, isobutyl-sulfinyl, sec-butylsulfinyl, tert-butylsulfinyl; C₆-C₁₀ arylsulfinyl such as phenylsulfinyl; C₁-C₄ alkylsulfonyl such as methylsulfonyl, ethylsulfonyl, propylsulfonyl, isopropylsulfonyl, butylsulfonyl, isobutylsulfonyl, sec-butylsulfonyl, tert-butylsulfonyl; C₆-C₁₀ arylsulfonyl such as phenylsulfonyl; C₁-C₄ alkoxysulfonyl such as methoxysulfonyl, ethoxysulfonyl, propoxysulfonyl, isopropyloxysulfonyl, butoxysulfonyl, isobutyloxysulfonyl, sec-butoxysulfonyl, tert-butoxysulfonyl; C₆-C₁₀ aryloxysulfonyl such as phenoxysulfonyl; amino; C₁-C₁₁ carboxyacylamino such as acetylamino, propionylamino, benzoylamino; mono- or di- C₁-C₄ alkylamino such as methylamino, ethylamino, propylamino, isopropylamino, butylamino, dimethylamino, diethylanimo; C₃-C₆ cycloalkylamino such as cyclohexylamino; C₆-C₁₀ arylamino such as anilino; trisubstituted silyl such as trimethylsilyl, tertbutyldimethylsilyl, triphenylsilyl, tert-butyl methoxyphenylsilyl; C₁-C₁₁ carboxylic acyl such as formyl, acetyl, benzoyl; a group selected from 3- to 6-membered heterocyclic groups containing 1-5 hetero atoms such as oxygen, sulfur or nitrogen, and condensed rings thereof, for example, such as 2- or 3-thienyl, 2- or 3-furyl, 1-,2- or 3-pyrrolyl, 2-, 3- or 4-pyridyl, 2-, 4- or 5-oxazolyl, 2-, 4- or 5-thiazolyl, 1-, 3-, 4- or 5-pyrazolyl, 1-, 2-, 4- or 5-imidazolyl, 3-, 4-or 5-isoxazolyl, 3-, 4- or 5-isothiazolyl, 1,2,3- or 1,2,4-triazolyl, 2-, 4- or 5-pyrimidinyl, benzothiazolyl, benzoxazolyl, triazinyl, oxilanyl, aziridinyl, pyrrolidinyl, piperidinyl, morpholinyl, benzimidazolyl, quinolyl, isoquinolyl; an amino acid residue such as, for example, alanyl, β--alanyl, arginyl, cystathionyl, cystyl, glycyl, histidyl, homoseryl, isoleucyl, lanthionyl, leucyl, lysyl, methionyl, norleucyl, norvalyl, ornithyl, prolyl, sarcosyl, seryl, threonyl, thyronyl, tyrosyl, varyl, etc.

The above substituent may also have one or more substituents that may be the same or different from one another in the same or different substitutable positions. In that case, the further substituents are equivalent to the aforesaid substituents. If two or more substituents are used, their two substituents taken together, may form a divalent group such as, for example, C₁-C₆ alkylene (e.g., methylene, ethylene, trimethylene, tetramethylene, propenylene), 3-oxapentamethylene, vinylene, benzylidene, methylenedioxy, 2-thiatrimethylene, oxalyl, malonyl, succinyl, maleoyl, phthaloyl, oxygen, sulfur, imino, azo, hydrazo and the like. If these substituents are divalent groups such as, for example, aryl, aralkyl, cycloalkyl, cycloalkenyl, aryloxy, arylthio, arylsulfinyl, arylsulfonyl, arylcarbamoyl, aryloxycarbonyl, aryloxysulfonyl, arylamino, cycloalkylamino, carboxylic acyl, carboxylic acylamino, tri-substituted silyl, and heterocyclic groups, 1 to 5 atoms of the substituents may further be substituted by one or more groups selected from the group consisting of halogen such as fluorine, chlorine, bromine and iodine; hydroxyl; nitro; cyano; C₁-C₄ alkyl such as methyl, ethyl, propyl, isopropyl, butyl, isobutyl, sec-butyl and tert-butyl; C₂-C₄ alkenyl such as vinyl and allyl; C₂-C₄ alkynyl such as, ethynyl, 2-propynyl and the like; phenyl; C₁-C₄ alkoxy such as methoxy, ethoxy and the like; phenoxy; C₁-C₄ alkylthio groups such as methylthio, ethylthio; and phenylthio group.

Further, if the substituents are groups selected from the group consisting of alkyl, alkenyl, alkynyl, alkoxy, alkylthio, alkylsulfinyl, alkylsulfonyl, alkylcarbamoyl, alkoxycarbonyl, alkoxysulfonyl, amino and alkylamino, 1 to 5 atoms of the substituent may further be substituted by one or more groups selected from the group consisting of halogen as mentioned above, hydroxyl, nitro; cyano; C₁-C₄ alkoxy and C₁-C₄ alkylthio groups.

As the divalent hydrocarbon group represented by R3, i.e., "the divalent group of linear or branched C₁-C₅ hydrocarbons which may have one or more unsaturated bonds", there are C₁-C₅ alkylene such ethylene, trimethylene, tetramethylene, pentamethylene; C₂-C₅ alkenylene such as vinylene, propenylene and the like.

In an embodiment of the present invention, the halogen atom in the above general formula (I) is chlorine atom.

In an embodiment of the present invention, the compound that deactivates α₁-protease inhibitor is at least one of N-chlorsuccinimides.

According to the present invention, there is further provided a drug for preventing vascular restenosis, comprising as an active constituent, a compound that deactivates α₂-macroglobulin.

As the above compound that deactivates α₂-macroglobulin, it is preferred to use a compound or a salt thereof, represented by the general formula:

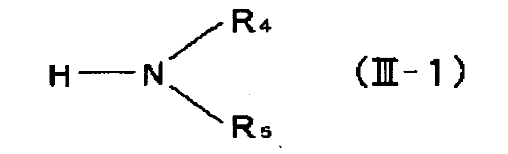

or (NH₄)ₚ(Y⁻)^{p} (III-2)

wherein R₄ is hydrogen or amino, R₅ is hydrogen, C₁-C₅ alkyl that may be being substituted or carbamoyl that may be being substituted, p is an integer ranging from 1 to 3, Y is an acid radical, R₄ and R₅ taken together, may form a group represented by the general formula: 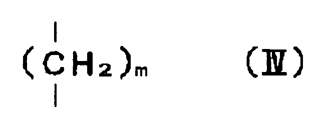 wherein m is an integer ranging from 1 to 6, in that case the α₂-macroglobulin-deactivating compound may be being polymerized by ring-opening polymerization as represented by the general formula (V):

-(CH₂CH₂NH)ₙ- (V)

wherein n is an integer ranging from 10 to 2500.

The amino group represented by R₄ in the above general formula includes, for example, a primary amino group, a secondary group or a ternary amino group.

The alkyl in unsubstituted or substituted alkyl represented by R₅ includes, for example, C₁-C₅ alkyl such as methyl, ethyl, propyl, isopropyl, butyl, isobutyl, sec-butyl, tert-butyl and pentyl.

The alkyl or carbamoyl represented by R5 may have one or more substituents, which may be the same or different with one another, in substitutable positions thereof. As preferred examples of the above substituents, there may be used, for example, C₃-C₁₀ cycloalkyl such as cyclopropyl, cyclobutyl, cyclopentyl and cyclohexyl; C₂-C₁₀ alkenyl such as vinyl, allyl, 2-methylallyl, 2-butenyl, 3-butenyl, 3-octenyl; C₂-C₁₀ alkynyl such as ethynyl, 2-propynyl, 3-hexynyl; C₃-C₁₀ cycloalkenyl such as cyclopropenyl, cyclobutenyl, cyclopentenyl and cyclohexenyl; C₆-C₁₀ aryl such as phenyl and naphthyl; C₇-C₁₁ aralkyl such as benzyl and phenylethyl; nitro; nitroso; hydroxyl; mercapto; cyano; oxo; thioxo; carbamoyl; mono or di- C₁-C₆ alkylcarbamoyl such as methylcarbamoyl, dimethylcarbamoyl; C₆-C₁₄ arylcarbamoyl such as phenylcarbamoyl; carboxyl; C₁-C₄ alkoxycarbonyl such as methoxycarbonyl, ethoxycarbonyl; C₆-C₁₄ aryloxy-carbonyl such as phenoxycarbonyl; sulfo ; halogen such as fluorine, chlorine, bromine and iodine; C₁-C₄ alkoxy such as methoxy, ethoxy, propoxy, isopropoxy, butoxy, isobutoxy, sec-butoxy, tert-butoxy; C₆-C₁₀ aryloxy such as phenoxy; C₁-C₄ alkylthio such as methylthio, ethylthio, propylthio, isopropylthio, butylthio, isobutylthio, sec-butylthio, tert-butylthio; C₆-C₁₀ arylthio such as phenylthio; C₁-C₄ alkylsulfinyl such as methylsulfinyl, ethylsulfinyl, propyl-sulfinyl, isopropylsulfinyl, butylsulfinyl, isobutyl-sulfinyl, sec-butylsulfinyl, tert-butylsulfinyl; C₆-C₁₀ arylsulfinyl such as phenylsulfinyl; C₁-C₄ alkylsulfonyl such as methylsulfonyl, ethylsulfonyl, propylsulfonyl, isopropyl-sulfonyl, butylsulfonyl, isobutylsulfonyl, sec-butylsulfonyl, tert-butylsulfonyl; C₆-C₁₀ arylsulfonyl such as phenylsulfonyl; C₁-C₄ alkoxysulfonyl such as methoxysulfonyl, ethoxysulfonyl, propoxysulfonyl, isopropyloxysulfonyl, butoxysulfonyl, isobutyloxysulfonyl, sec-butoxysulfonyl, tert-butoxysulfonyl; C₆-C₁₀ aryloxysulfonyl such as phenoxysulfonyl; amino; C₁-C₁₁ carboxyacylamino such as acetylamino, propionylamino, benzoylamino; mono- or di- C₁-C₄ alkylamino such as methylamino, ethylamino, propylamino, isopropylamino, butylamino, dimethylamino, diethylanimo; C₃-C₆ cycloalkylamino such as cyclohexylamino; C₆-C₁₀ arylamino such as anilino; trisubstituted silyl such as trimethylsilyl, tertbutyldimethylsilyl, triphenylsilyl, tert-butyl methoxyphenylsilyl; C₁-C₁₁ carboxylic acyl such as formyl, acetyl, benzoyl; a group selected from 3- to 6-membered heterocyclic groups containing 1-5 hetero atoms such as oxygen, sulfur or nitrogen, and condensed rings thereof, for example, such as 2- or 3-thienyl, 2- or 3-furyl, 1-,2- or 3-pyrrolyl, 2-, 3- or 4-pyridyl, 2-, 4- or 5-oxazolyl, 2-, 4- or 5-thiazolyl, 1-, 3-, 4- or 5-pyrazolyl, 1-, 2-, 4- or 5-imidazolyl, 3-, 4-or 5-isoxazolyl, 3-, 4- or 5-isothiazolyl, 1,2,3- or 1,2,4-triazolyl, 2-, 4- or 5-pyrimidinyl, benzothiazolyl, benzoxazolyl, triazinyl, oxilanyl, aziridinyl, pyrrolidinyl, piperidinyl, morpholinyl, benzimidazolyl, quinolyl, isoquinolyl; an amino acid residue such as, for example, alanyl, β-alanyl, arginyl, cystathionyl, cystyl, glycyl, histidyl, homoseryl, isoleucyl, lanthionyl, leucyl, lysyl, methionyl, norleucyl, norvalyl, ornithyl, prolyl, sarcosyl, seryl, threonyl, thyronyl, tyrosyl, varyl, etc.

The above substituent may also have one or more substituents that may be the same with or different from the other, in the same or different substitutable positions. In that case, the further substituents are equivalent to the aforesaid substituents. If two or more substituents are used, their two substituents taken together, may form a divalent group such as, for example, C₁-C₆ alkylene (e.g., methylene, ethylene, trimethylene, tetramethylene, propenylene), 3-oxapentamethylene, vinylene, benzylidene, methylenedioxy, 2-thiatrimethylene, oxalyl, malonyl, succinyl, maleoyl, phthaloyl, oxygen, sulfur, imino, azo, hydrazo and the like. If these substituents are divalent groups such as, for example, aryl, aralkyl, cycloalkyl, cycloalkenyl, aryloxy, arylthio, arylsulfinyl, arylsulfonyl, arylcarbamoyl, aryloxycarbonyl, aryloxysulfonyl, arylamino, cycloalkylamino, carboxylic acyl, carboxylic acylamino, tri-substituted silyl, and heterocyclic groups, 1 to 5 atoms of the substituents may further be substituted by one or more groups selected from the group consisting of halogen such as fluorine, chlorine, bromine and iodine; hydroxyl; nitro; cyano; C₁-C₄ alkyl such as methyl, ethyl, propyl, isopropyl, butyl, isobutyl, sec-butyl and tert-butyl; C₂-C₄ alkenyl such as vinyl and allyl; C₂-C₄ alkynyl such as, ethynyl, 2-propynyl and the like; phenyl; C₁-C₄ alkoxy such as methoxy, ethoxy and the like; phenoxy; C₁-C₄ alkylthio groups such as methylthio, ethylthio; and phenylthio group.

Further, if the substituents are groups selected from the group consisting of alkyl, alkenyl, alkynyl, alkoxy, alkylthio, alkylsulfinyl, alkylsulfonyl, alkylcarbamoyl, alkoxycarbonyl, alkoxysulfonyl, amino and alkylamino, 1 to 5 atoms of the substituent may further be substituted by one or more groups selected from the group consisting of halogen as mentioned above, hydroxyl, nitro; cyano; C₁-C₄ alkoxy and C₁-C₄ alkylthio groups.

The acid radical represented by Y includes chloro, carbonate, sulfate, nitrate and borate groups. Preferred acid radical is sulfate.

In a preferred embodiment of the present invention, the compound that deactivates α₂-macroglobulin includes compounds containing an amino group. Such an amino-containing compound includes, for example, ammonia, amine compounds and salts thereof, urea, hydrazine and salts thereof, ammonium salts, imine and salts thereof, tobramycin, polylysines and lysozyme chloride, and the compound used is at least one compound selected from the group consisting of these compounds. When imine is used as the compound that deactivates α₂-macroglobulin, it is preferred to use polyethylene imine having a molecular weight ranging from 500 to 100000, measured by gel filtration technique. In another embodiments, ammonium sulfate is employed as the compound that deactivates α₂-macroglobulin.

The above compound that deactivates α₂-macroglobulin may constitute a drug for prevention of vascular restenosis in combination with the above compound that deactivates α₁-protease inhibitor.

In another aspect of the present invention, there is provided an implantable intravascular device coated with a drug for prevention of vascular restenosis that comprises, as an active constituent, a compound that deactivates α₁-protease inhibitor and/or a compound that deactivates α₂-macroglobulin. As an implantable intravascular device, it is preferred to use a stent.

### Best Mode for Carrying Out the Invention

Firstly, the explanation will be made on a drug for prevention of vascular restenosis according to the present invention.

According to the present invention, there is no limit to the compound that deactivates α₁-protease inhibitor, provided that the compound is able to deactivate the α₁-protease inhibitor. Since an active center of the α₁-protease inhibitor is considered to be blocked by an oxide, it is sufficient for the compound that deactivates α₁-protease inhibitor to have a behavior to attack the oxide. Thus, it is preferred to use a compound having partial structure represented by formula: 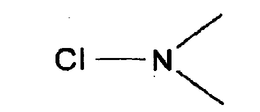 Examples of the compounds having such a partial structure are cyclic imides or salts thereof derived from acyl of dibasic acids such as N-chloroxalimido, N-chlormalonimido, N-chlorsuccinimide, N-chlorphthalimide, N-chlormaleimide, N-chlorglutarimide; chloramine compounds obtained by replacing at least one hydrogen atom of ammonia with chlorine atom, or salts thereof. It is preferred to employ chlorsuccinimide. These compounds may be used singly or in combination.

Further, there is no limit to the compound of the present invention that deactivates α₂-macroglobulin, provided that the compound is able to deactivate α₂-macroglobulin. Since it is considered that α₂-macroglobulin is deactivated by the formation of a covalent bond between glutamic acid present in an active center of α₂-macroglobulin and an amino group, it is preferred to use a compound having an amino group. As such a compound, there may be used ammonia; amine compounds or salts thereof having C₁-C₅ alkyl replaced for one hydrogen atom of ammonia; urea having a carbamoyl group replaced for one hydrogen atom of ammonia; hydrazine or salts thereof having an amino group replaced for one hydrogen atom of ammonia; ammonium salts; and cyclic secondary amines having a divalent hydrocarbon replaced for two hydrogen atoms of ammonia, i.e., imines or salts thereof.

As the amine compound, it is preferred to use primary amine, in particular, methylamine or ethylamine, especially, methylamine. These amines may be used in the form of a salt thereof. As an ammonium salt, there may be used those such as, for example, ammonium hydrochloride, ammonium sulfate, ammonium carbonate, ammonium hydrogen carbonate, ammonium nitrate, ammonium borate. The most preferred ammonium salt is ammonium sulfate. The cyclic secondary amines having a divalent hydrocarbon replaced for two hydrogen atoms of ammonia includes imines represented by the general formula: 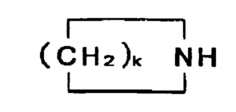 wherein k is an integer ranging from 1 to 6, such as, for example, ethylene imine, pyrrolidine and piperidine. A preferred imine is ethylene imine. The ethylene imine may be used in the form of polyethylene imine obtained by ring opening polymerization and expressed by the general formula:

-(CH₂CH₂NH)ₙ- (V)

wherein n is an integer ranging from 10 to 2500. In that case, preferred ethylene imine is polyethylene imine having a molecular weight ranging from 500 to 100000, measured by gel filtration technique. These drugs may be used singly or in combination with two or more drugs.

Further, if the C1-C5 alkyl with a substituent has any further substituent, examples of such a compounds include tobramycin polylysine, elcatonin and lysozyme chloride.

The compound that deactivates α₁-protease inhibitor and the compound that deactivates α₂-macroglobulin may be administered in the form of bulk powder, but they are generally administered in the form of preparations prepared by mixing the component with any component selected from the group consisting of additives, fillers (e.g., calcium carbonate, lactose, crystallized cellulose, starches, porous materials, etc.), disintegrants(e.g., low-substituted hydroxypropylcellulose, carboxymethylcellulose calcium, etc.), binders(e.g., dextrin, gelatine, pullulans, etc.), lubricants(e.g., magnesium stearate, calcium stearate, etc.), coating agents(e.g., methyl cellulose, ethyl cellulose and the like), surfactants(e.g., polyoxyethylene hydrogenated castor oils, polysorbate, etc.), elasticizer(e.g., triethyl citrate, triacetin, etc.), antioxidant agents(e.g., citric acid, α-tocopherol, etc.) and preservative(e.g., parabens and the like), and then making a suitable amount of the resultant mixture to a desired dosage form.

The dosage form includes, for example, tablets, balls, capsules, granules, subtle granules, powder, syrup, injectable solutions, suspensions, fat emulsion preparations, inhalants, ointment, etc., any of which may be prepared in the ordinary manner, for example, as described in Japanese Pharmacopoeia. Among them, it is preferred to use a dosage form suitable for parenteral and topical administration such as injectable solutions, suspensions and fat emulsion preparations.

A more preferred process is administration of a drug to an affected area directly with a drug delivery catheter or an implantable intravascular device coated with the drug. The most preferred process is placement of a stent coated with an drug for prevention of restenosis in the affected area. A process employing a stent is simple and easy in insertion, so that it requires no specialized technique and is short in time required for implantation of the stent in the affected area. Further, the stent coated with an drug for prevention of restenosis is locally implanted in the affected area, a dosage of the drug for regular adult is small and causes no side issues such as side effects.

Further, the dosage of drug for prevention of restenosis according to the present invention varies with administration routes, a patient's symptoms, age or weight. It is preferred to administer the drug such that a local concentration of the compound at the narrowed area of the vessel ranges from 1 to 100 mM. The concentration less than 1 mM does not have much effect on prevention of vascular restenosis, while the concentration exceeding 100 mM may cause side effects. The local concentration of the active component of the drug for prevention of vascular restenosis ranges from, preferably, 70 to 90 mM and, most preferably, is about 80 mM. To keep the local concentration, it is preferred to administer the drug for prevention of restenosis to the area parenterally and topically. This may be done, for example, by placement of an implantable intravascular device, especially a stent, coated with the drug for prevention of vascular restenosis of the present invention in the affected area, to achieve sustained-release of the drug.

Respective constituents and preparation process of the drug for prevention of vascular restenosis according to the present invention are described in detail in Japanese patent publications JP2000-249709(A), JP2000-95706(A) and Japanese Patent No. 3146009, which are hereby incorporated by reference.

Though the drug for prevention of vascular restenosis of the present invention has been explained as above, the vascular restenosis can be effectively prevented by use of a drug delivery balloon or a stent coated with the drug for prevention of vascular restenosis of the present invention to perform local administration of the drug.

Next, the explanation will be made on the implantable intravascular device of the present invention, making reference to a stent as an example.

A stent which is a preferred embodiment of implantable intravascular device of the present invention is characterized by the fact that a surface of the stent is coated with a drug for prevention of vascular restenosis. In general, the stent has a cylindrical form, has a coiled body portion or a meshed body portion having plural slots. But the stent is not limited to these configurations and may take any configuration, provided that it can maintain a size of the lumen after widening the narrowed area of the vessel. Also, the stent may have an expansion mechanism of a self-expandable type or a balloon expandable type.

Suitable materials for the stent includes metals such as stainless steel, titanium, tantalum, alminum, tungsten and nitinol; plastics such as polyamides, polyethylene, polypropylene and hardly-hydrolyzable polyether polyurethane. However, there is no limit to the material for the stent, provided that the material is biocompatible and has been proved as a material for medical use.

Examples of the stents includes Palmaz-Schatz (trademark) stent of Johnson & Johnson, MULTI-LINK (trademark) stent of Advanced cardiovascular systems, Cordis (trademark) stent of Cordis Corporation and Microstent-GFX (trademark) of Medotronic Arterial Vascular Enginerering, Inc., which can be used with good results.

The stent of the present invention may be obtained by applying a coating mixture containing the drug for prevention of vascular restenosis of the present invention to the above stent in the conventionally known manner. Methods for coating to stents are described in Japanese patent unexamined publications JP2000-350786 A and JP2000-51367A, which are hereby incorporated by reference. However, these coating methods can be applied to implantable intravascular devices other than stent. The following is explanation of a method for coating a drug for prevention of vascular restenosis to the stent.

The drug-coated stent may be obtained by (1) a method comprising steps of mixing a drug for prevention of vascular restenosis with any one of various polymers to prepare a coating mixture, and then spraying the resultant mixture on a stent or dipping the stent into the resultant mixture, or (2) a method comprising steps of providing a hydrophilic coating of a polymer on surface of a stent and then spraying the drug for prevention of vascular restenosis on a stent or dipping the stent into the drug for prevention of vascular restenosis. The drug for prevention of vascular restenosis is used after formulation to a suitable dosage form such as a solution, powder or the like, to make it easy to mix the drug with any one of the various polymers. If a powder type drug for prevention of vascular restenosis is mixed with polymer, it is necessary to sufficiently reduce granularity (primary particles and secondary particles such as aggregates or lumps) to provide the stent with smooth surfaces after coating as well as to prevent slots of the stent from being clogged with the drug.

The above coating mixture may includes one or more additives such as, for example, diluents, carriers, fillers, stabilizing agents and the like. For example, one or more hydrophilic polymers selected from biocompatible hydrophilic polymers may be added to a biocompatible hydrophobic coating agent to modify release profile of the drug. Also, one or more hydrophobic polymers may be added to a biocompatible hydrophilic coating agent to modify release profile of the drug. For example, the release profile may be modified by incorporating a hydrophilic polymer selected from the group consisting of polyethylene oxide, polyvinyl pyrrolidone, polyethylene glycols, carboxymethylcellulose, hydroxymethylcellulose and combination thereof into an aliphatic polyester coating agent. An appropriate amount of the hydrophilic polymer added to the hydrophobic coating agent or that of hydrophobic polymer added to the hydrophilic coating agent is determined according to the release profile of the drug for prevention of vascular restenosis in vitro or in vivo.

There is no limit to the polymers used in the present invention if they are biocompatible. Examples of the polymers are polyamides, polyolefines (including polypropylene, polyethylene), nonabsorbable polyesters (including polyethylene terephthalate and the like) and biologically absorbable aliphatic polyesters (lactic acid, glycolic acid, lactide, glycollide, p-dioxane, trimethylenecarbonates, ε-caprolactam, homopolymers thereof and copolymers derived from a mixture of these materials).

Further, the polymer may be mixed with a highly flexible, high molecular elastomer to provide coating layers with flexibility. The elastomers that can be used include, for example, various elastomers of polystyrene, polyolefine, polyesters, polyamides, silicone, polyurethane, fluorinated elastomer, natural rubber, and copolymers or polymer alloys of these materials. Among them, the most preferred material is a segmented polyurethane polymer film with a high flexibility and a high strength. It is preferred to use segmented polyurethane polymer films including flexible polyether soft-segments and hard segments of aromatic and urea bond-rich portions, the soft segments and hard segments forming a microstructure based on phase separation. Such segmented polyurethane polymer films have sufficient strength and are excellent in antithrombogenicity. In other words, the segmented polyurethane polymer films are biocompatible and can be expanded sufficiently without causing rupture.

An amount of the drug for prevention of vascular restenosis to be coated on the stent varies with the administration route, a patient's symptoms, age or weight. The quantity consumed of the drug for prevention of vascular restenosis ranges from about 0.001 w/v% to about 70 w/v%, more generally, from about 0.001 w/v% to about 60 w/v% and, most generally, from about 0.001 w/v% to about 45 w/v% with respect to the total amount of the coating mixture.

According to the present invention, the stent explained above makes it possible to provide low invasive means for prevention of vascular restenosis in a short time after coronary interventions as well as to effectively prevent the vascular restenosis, thus making it possible to reduce mental and physical pains of the patient.

It is to be noted that the coronary interventions with possibility of postoperative vascular restenosis, to which the drug for prevention of vascular restenosis of the present invention and implantable intravascular device coated therewith are applied, include, without being limited thereto, Directional Coronary Atherectomy (DCA), Transluminal Extraction Catheter(TEC), rotating atherectomy coronary angioplasty (Rotablator) and Laser Angioplasty, in addition to the aforesaid PTCA and stent placement.

### BRIEF DESCRIPTION OF DRAWINGS

Fig. 1 is a graph illustrating relationship between decreasing index for vascular restenosis rate and concentrations of α₁-protease inhibitor, α₂-macroglobulin and α₁-antichymotrypsin in serum;
Fig. 2 is a graph illustrating relationship between presence or non-presence of vascular restenosis and concentration of α₂-macroglobulin in serum for both diabetic patient group and nondiabetic patient group;
Fig. 3 is a graph illustrating relationship between a concentration of N-chlorsuccinimide in fibrin and migration incidence of vascular plain muscles;
Fig. 4 is a graph illustrating Intima-Media Thickness (I/M ratio) for an N-chlorsuccinimide-treated group and a control group (right common iliac artery 3-times injury model);
Fig. 5 is a graph illustrating effects of methylamine, ethylamine, polyethylene imine with molecular weight of 600 to 10000, tobramycin, ammonia, lysozyme chloride and polylysine on deactivation of α₂-macroglobulin.

### EXAMPLES

The invention will be specifically explained below with reference to experimental tests.

In the examples, The test of significant difference on results for respective experimental tests was carried out by Pearson text with a SAS (trademark) system Ver. 6.12, made by SAS Institute Japan, unless otherwise specified. Symbols in experimental tests respectively denote the followings, unless otherwise stated. a1PI: α₁-protease inhibitor, a2M: α₂-macroglobulin, a1ACT: α₁-antichymotrypsin, p: rate of rejection, R: correlation coefficient, M: mol concentration (mol/L), mM: millimol concentration (mmol/L), mg: milligram, w/v%: weight/volume percentage, ISR: In-stent restenosis.

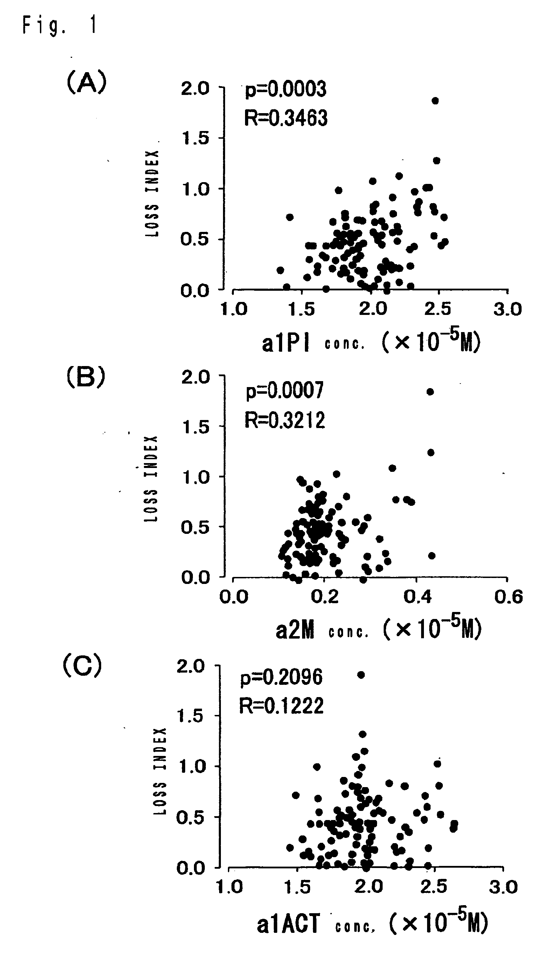

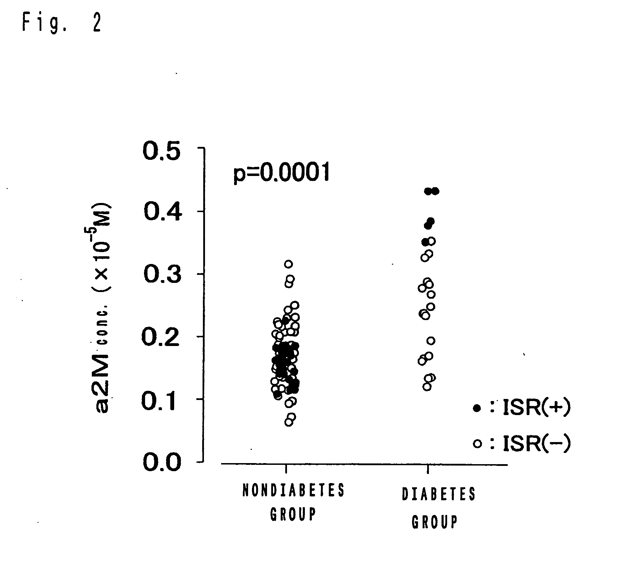

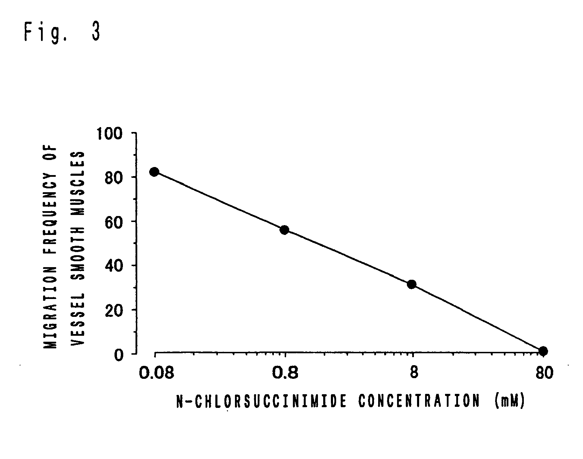

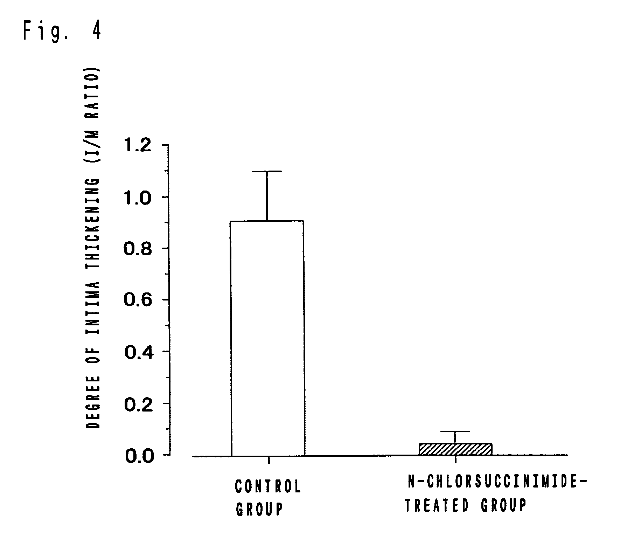

The followings are explicative of experimental results on case patients received stenting, which reveals that α₁-protease inhibitor and α₂-macroglobulin are ISR related factors as mentioned below.
[EXPERIMENT 1] Influences of concentrations of α₁-protease inhibitor and α₂-macroglobulin in serum on reduction of vascular lumen diameter
   The objects of experiments were case patients with coronary artery diseases requiring coronary-artery intervention, who are de novo lesion and have received stent placements.
   In order to prevent the patients from acute and subacute thrombotic occlusion, pretreatments were performed by administering aspirin (81 mg or 162 mg) and ticlopidine (200 mg) or cilostazole (200 mg) for 7 days prior to the therapy. There is no limit to the length of lesion and the selection of a stent was left to the discretion of the operator. Stenting was succeeded in 91 cases and 110 lesions. Among them, the 107 lesions were subjected to follow-up coronary angiography after 6 months. These 107 lesions were tested for verifications of relationship between concentrations of α₁-protease inhibitor, α₂-macroglobulin in blood and inner diameter of the vessel. The inner diameter of the blood vessel was measured before and after stent placement and after 6 months with a cardiovascular analysis software, QCA-CMS of CMS, and loss index which represents the level of vascular restenosis was determined from acute gain and late loss.
   Blood serums were collected from the respective case patients after 24 hours of the therapy and subjected to the nephelometric analysis to determine concentrations of α₁-protease inhibitor and α₂-macroglobulin along with that of α₁-antichymotrypsin as the control material. The measurements were carried out in an orderly manner by using measurement kits made by Dade Behring Marburg for α₁-protease inhibitor and α₂-macroglobulin and measurement kits made by Hoechst Japan for α₁-antichymotrypsin.
   Results are shown in Fig. 1. In Fig. 1, (A) denotes the relationship between the concentrations of α₁-protease inhibitor in serum and loss index, (B) denotes the relationship between the concentrations of α₂-macroglobulin in serum and loss index, and (C) denotes the relationship between the concentrations of α₁-antichymotrypsin in serum and loss index. In each diagram, a vertical axis indicates the loss index, while a horizontal axis indicates the concentration of respective factors in serum.
   As shown in Fig. 1, the mean values of concentrations of α₁-protease inhibitor, α₂-macroglobulin and α₁-antichymotrypsin in serum were fallen in the normal range. As can be seen from the results shown in Fig. 1, there was positive correlation between the loss index and α₁-protease inhibitor or α₂-macroglobulin, but there was no correlation between α₁-antichymotrypsin and the loss index. In other words, it was confirmed that there is statistical correlation between α₁-protease inhibitor or α₂-macroglobulin and ISR.
   Further, Fig. 2 shows the relationship between the concentration of α₂-macroglobulin in serum and ISR, obtained by separating the case patients into a diabetic group and a nondiabetic group. In that figure, a black circle denotes the presence of ISR, while a white circle denotes no presence of ISR. The presence or absence of ISR was determined by measurement of the inner diameter of the blood vessel with a quantitative coronary angiography, and more than 50 % diameter stenosis was judged to be ISR.
   As shown in Fig. 2, the concentrations of α₂-macroglobulin in serum for the diabetic group are higher than that of the nondiabetic group. The data for the nondiabetic group have no correlation between concentration of α₂-macroglobulin in serum and ISR. In contrast therewith, the data for the diabetic group have a clear correlation between concentration of α₂-macroglobulin in serum and ISR since the increase in concentration of α₂-macroglobulin in serum have a high tendency to increase the occurrence of ISR.
   For example, in the case patients of the diabetic group, the concentration of α₂-macroglobulin higher than the normal upper limit of 0.35×10⁻⁵ M causes a 75 % occurrence of ISR, while the concentration of α₂-macroglobulin lower than 0.35×10⁻⁵ M provides a 0% occurrence of ISR. Thus, in the case patients of the diabetic group, it was revealed that the concentration of α₂-macroglobulin in serum has a considerably strong correlation with the occurrence of ISR.
   In other words, it can be said that the vascular restenosis preventing agent according to the present invention is a highly effective drug for diabetic patients.
   Accordingly, it will be seen that the prevention of vascular restenosis can be expected by inhibiting α₁-protease inhibitor and α₂-macroglobulin.
   Although serum factors associated with the vascular restenosis have been studied by investigators in the world, at the present moment there is no report on the serum factor that can be revealed to have such a correlation therewith.
   The next is an explanation on test results showing pharmacologic effects of the compound (I).
[EXPERIMENT 2] Influences of N-chlorsuccinimide in fibrin on the migration frequencies of vascular smooth muscles
   The vascular smooth muscles were collected from human vital arteries and established HNB18E6E7 was used as cell lines. The vascular smooth muscles were incubated in 6-well plates to produce confluent cultures. The resultant supernatant fluids were washed three time with warmed phosphate buffer solution (PBS) and softly added with 3 mg/mL of fibrinogen and 0.8 mL of Waymouth culture solution including 10 w/v% human serum. Then, the supernatant fluids were respectively added with N-chlorsuccinimide to adjust the final concentration of N-chlorsuccinimide to 0.08 mM, 0.8 mM, 8 mM and 80mM, and gelled by addition of thrombin. The resultant gelled supernatants were incubated at 37 ° C for 24 hours, and the numbers of the smooth muscle cells migrated into the gel was counted with a phase contrast microscope. For each concentration, the experiments were carried out to make n = 3. Results are shown in Fig. 3. The ordinate denotes the frequencies of migration of vascular smooth muscles into the gel, while the abscissa denotes the concentration of N-chlorsuccinimide.
   As illustrated in Fig. 3, the frequency of migration of the vascular smooth muscles decreases apparently with increasing concentration of N-chlorsuccinimide, and the number of migrating vascular smooth muscles vanished completely at 80 mM. The results proved that N-chlorsuccinimide that is an inhibitor of α₁-protease inhibitor has a beneficial effect on the prevention of vascular restenosis.
   Although N-chlorsuccinimide was used in the above experiment, since the active centers of α₁-protease inhibitor are protected by oxides, it is possible to use any other compound having chlorine atom and an oxidizing ability and including a partial structure represented by the formula: 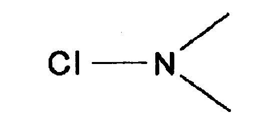 Explanation will be made on experimental results proving medicinal values of the compound(I).
[EXPERIMENT 3] Influences of N-chlorsuccinimide on neointimal formation in vascular injury model
   There were used a group of animals consisted of 5 (five) N2W male rabbits aged 2 months. Vascular injury models were prepared by introducing a Fogarty catheter, Model e-060-2F, made by Baxter corporation (that is an arterial embolectomy balloon catheter) into the right common iliac artery through the right femoral artery under pentobarbital anesthesia, and then abrading the right common iliac artery 3 times under conditions of the balloon being inflated (balloon pressure: 0.5 - 1.0 atm). After injuring the vascular endothelium, hydrophilic balloons made by Boston Scientific Corporation, of an N-chlorsuccinimide-treated group prepared by immersing the balloons in 80 mM N-chlorsuccinimide, and of a control group prepared by immersing the balloons in PBS, were respectively inflated in the right common iliac arteries to locally administer the drug to each injured area.
   After 4 weeks from the treatment, the arteries were surgically removed, fixed with 10w/v% neutral buffered formalin fixative and then sectioned to prepare arterial ring tissue sections. After preparing elastic fiber stained tissue samples by EVG (Elastic Van Gieson) staining, the samples were subjected to measurements of intimal area (I) and medial area (M) to determine their ratio (I/M) as the degree of intimal thickening. Results are shown in Fig. 4, in which the ordinate denotes the degree of intimal thickening (I/M ratio), and the abscissa denotes the treated group names.
   As illustrated in Fig.4, in the group treated with N-chlorsuccinimide, the neointimal hyperplasia is considerably inhibited. It is proved that the compound is effective against the vascular restenosis.
   Explanation will be made on experimental results proving medicinal values of the compound (III-1).
[EXPERIMENT 4] Effects of methylamine, ethylamine, polyethyleneimines with molecular weights of 600 and 10000, tobramycin, ammonia, lysozyme chloride and polylysine on deactivation of α₂-macroglobulin

Utilizing the facts that trypsin is inhibited by α₂-macroglobulin and that trypsin causes yellow coloring of a coloring matrix S2222 resulting from decomposition thereof, verification was carried out for effects of methylamine, ethylamine, polyethyleneimines with molecular weights of 600 and 10000, tobramycin, ammonia, lysozyme chloride and polylysine on deactivation of α₂-macroglobulin. A concrete method will be given below.

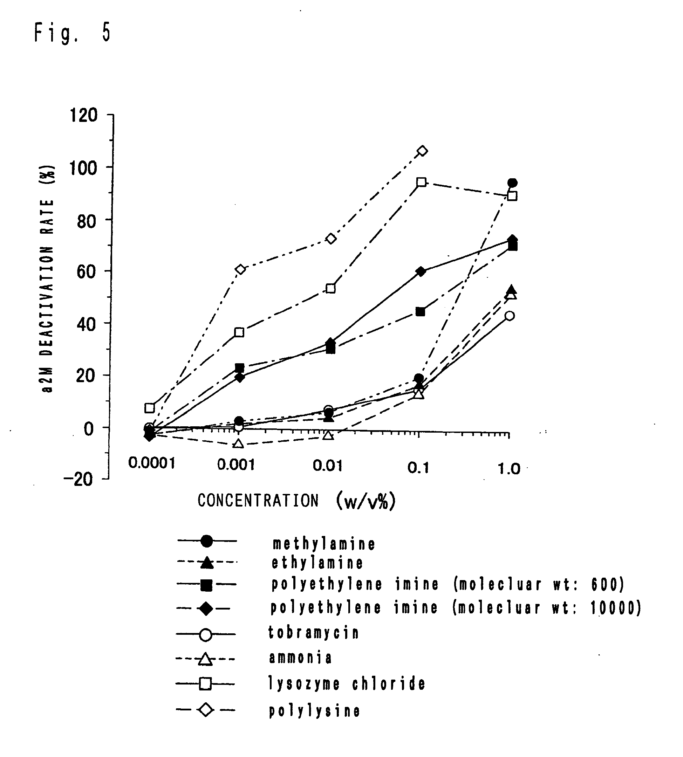

Firstly, each of methylamine, ethylamine, polyethyleneimines with molecular weights of 600 and 10000, tobramycin, ammonia, lysozyme chloride and polylysine was diluted to 0.0001 w/v%, 0.001w/v%, 0.01 w/v%, 0.1 w/v% and 1 w/v% to prepare a series of diluted solutions as sample solutions. 50 µL of each sample solution was added with 50 µL of 2.5×10⁻⁴w/v% α₂-macroglobulin and left for 5 minutes at room temperature. Then, the sample solution was added with 50 µL of a 5 w/v% trypsin EDTA solution, left for 5 minutes at room temperature, and then added with 10 µL of the coloring matrix S2222. After leaving the resultant sample solution for 20 minutes at room temperature, the sample solution was added with 50 µL of 10 w/v% acetic acid solution to stop reactions, and then subjected to measurement of absorption of light with a wave length of 405 nm, using 50 mM tris(hydroxymethyl)aminomethane buffer solution of pH 8.0 as the blank. The dilution of all the samples and agents was carried out by a 50 mM tris(hydroxymethyl)aminomethane buffer solution of pH 8.0. Results are shown in Fig. 5, in which the ordinate denotes the deactivation rate of α₂-macroglobulin, and the abscissa denotes the concentrations of respective compounds.

As shown in Fig. 5, in each compound, increasing concentration thereof deactivates α₂-macroglobulin. Polylysine and lysozyme chloride provide 100% deactivation rate of α₂-macroglobulin at the concentration of 0.1 w/v%, while methylamine provides about 100% deactivation rate of α₂-macroglobulin at the concentration of 1 w/v%. Further, polymethylene imine with molecular weights of 10000 provides about 60% deactivation rate of α₂-macroglobulin at the concentration of 0.1 w/v%. Since each compound has the amino group, it is considered that α₂-macroglobulin is deactivated by the formation of covalent bonds between the amino group and active centers of α2-macroglobulin.

The vascular restenosis preventing agent according to the present invention and the implantable intravascular device coated therewith deactivate α₁-protease inhibitor and α₂-macroglobulin, which are factors being closely related to generation of vascular restenosis, thus making it possible to effectively prevent and treat the restenosis of the blood vessel expanded by coronary artery intervention. The above restenosis preventing agents provide sufficient effects even at low concentrations, have very little side effects, and in particular work on diabetic patients effectively. In addition, in case that the above restenosis preventing agents is locally applied to the affected area with an implantable intravascular device, in particular, a stents coated with the agent, the effects of the agent increase greatly and a risk of side effects is considerably lowered.

Also described is:
(1) A drug for preventing vascular restenosis, containing as an active constituent, a compound that inactivates α₁-proteases inhibitor.
(2) The drug for preventing vascular restenosis according to (1), wherein the compound that inactivates α₁- protease inhibitor is a compound or a salt thereof, represented by the general formula: 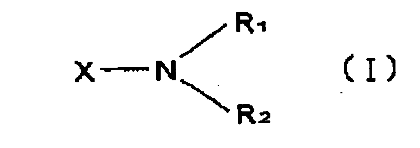 wherein X is halogen, R₁ is hydrogen or an alkaline metal R₂ is hydrogen or arylsufonyl which may be being substituted, R₁ and R₂ taken together, can form a group represented by the general formula:

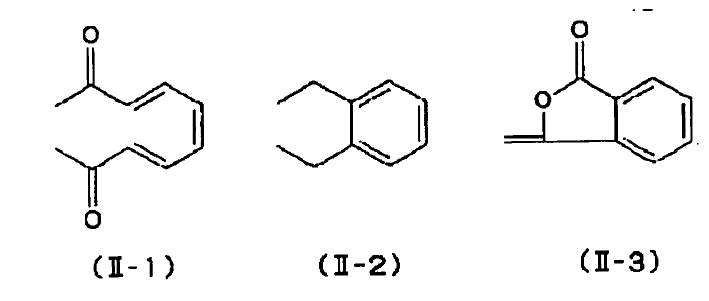

   or -CO-R₃-CO- (II-4)

   wherein R₃ is a divalent group of a linear or branched C₁-C₅ hydrocarbon which may have one or more unsaturated bonds, or a divalent bonding group.
(3) The drug for preventing vascular restenosis according to (2), wherein the halogen atom is a chlorine atom.
(4) The drug for preventing vascular restenosis according to (1), wherein the compound that inactivates α₁- protease inhibitor is N-chlorsuccinimide.
(5) An implantable intravascular device coated with a drug for preventing vascular restenosis defined in (1).
(6) The implantable intravascular device according to (5), wherein said device is a stent.
(7) A drug for presenting vascular restenosis, containing as an active constituent, a compound that inactivates α₂-macroglobulin.
(8) A drug for presenting vascular restenosis, according to (7), wherein the compound that inactivates α₂- macroglobulin is a compound or a salt thereof, said compound being represented by the general formula:

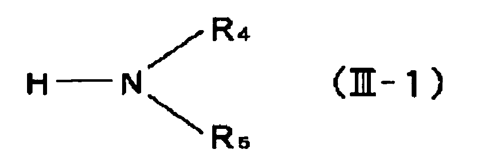

   or (NH₄)ₚ(Y⁻)^{p} (III-2)

   wherein R₄ is hydrogen or amino R₅ is hydrogen, C₁-C₅ alkyl that may be being substituted or carbamoyl that may be being substituted, p is an integer ranging from 1 to 3, Y is an acid radical, R₄ and R₅ taken together, may form a group represented by the general formula: 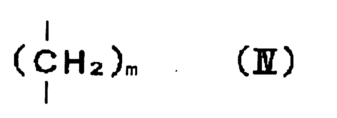 wherein m is an integer ranging from 1 to 6, in that case the α₂- macroglobulin-inactivating compound may be being polymerized by ring-opening polymerization as represented by the general formula: (V):

   -(CH₂CH₂NH)ₙ- (V)

   wherein n is an integer ranging from 10 to 2500.
(9) The drug for presenting vascular restenosis according to (8), wherein the compound that inactivates α₂-macroglobulin is at least one compound selected from the group consisting of ammonia, methylamine, ethylamine, salts thereof, urea, tobramycin, polylysines and lysozyme chloride.
(10) The drug for presenting vascular restenosis according to (8), wherein the compound that inactivates α₂-macroglobulin is polyethylene imine, preferably having a molecular weight ranging from 500 to 100000, measured by gel filtration technique.
(11) The drug for presenting vascular restenosis according to (8), wherein the compound that inactivates α₂-macroglobulin is ammonium sulfate.
(12) A drug for presenting vascular restenosis according to (7), further comprising a compound that inactivates α₁-protease inhibitor in combination with said compound.
(13) An implantable intravascular device coated with a drug for preventing vascular restenosis defined in (7) or (12).
(14) An implantable intravascular device coated device according to (13), wherein said device is a stent.

## Claims

1. A composition for preventing vascular restenosis, containing as an active constituent, a compound that inactivates α₂- macroglobulin.

2. The composition for preventing vascular restenosis according to claim 1, wherein the compound that inactivates α₂- macroglobulin is a compound or a salt thereof, said compound being represented by the general formula: 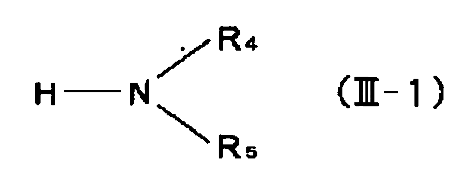
or (NH₄)ₚ(Y⁻)^{p} (III-2)
wherein R₄ is hydrogen or amino, R₅ is hydrogen, C₁-C₅ alkyl that may be being substituted or carbamoyl that may be being substituted, p is an integer ranging from 1 to 3, Y is an acid radical, R₄ and R₅ taken together, may form a group represented by the general formula: 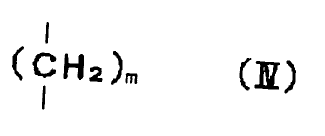 wherein m is an integer ranging from 1 to 6, in that case the alpha 2-macroglobulin-inactivating compound may be being polymerized by ring-opening polymerization as represented by the general formula (V):
(V): -(CH₂CH₂NH)ₙ- (V)
wherein n is an integer ranging from 10 to 2500.

3. The composition for preventing vascular restenosis according to claim 2, wherein the compound that inactivates alpha 2-macroglobulin is at least one compound selected from the group consisting of ammonia, methylamine, ethylamine, salts thereof, urea, tobramycin, polylysines and lysozyme chloride.

4. The composition for preventing vascular restenosis according to claim 8, wherein the compound that inactivates α₂- macroglobulin is polyethylene imine, preferably having a molecular weight ranging from 500 to 100000, measured by gel filtration technique.

5. The composition for preventing vascular restenosis according to claim 2, wherein the compound that inactivates α₂-macroglobulin is ammonium sulfate.

6. The composition for preventing vascular restenosis according to claim 1, further comprising a compound that inactivates alpha 1-protease inhibitor in combination with said compound.

7. An implantable intravascular device coated with a drug for preventing vascular restenosis defined in any one of claims 1 to 7.

8. The implantable intravascular device according to claim 7, wherein said device is a stent.

9. Use of a compound that inhibits α₂-macroglobulin in the manufacture of a medicament for the inhibition of vascular restenosis.

10. Use according to claim 9 wherein the compounds is as defined in any use of claims 1 to 6.

11. A pharmaceutical composition comprising a compound as defined in any one of claims 1 to 6.

12. The composition according to any one of claims 1 to 6, the implantable intravascular device of claim 6 or 7, the use of claim 9 or 10 or the pharmaceutical composition of claim 11 formulated for use in a diabetic individual.
